# EUROPEAN PATENT SPECIFICATION

(11) **EP 1 517 660 B1**
(45) Date of publication and mention of the grant of the patent: **24.08.2011**
(21) Application number: 03756228.7
(22) Date of filing: 28.05.2003
(51) Int. Cl.: A61F 13/53

(54) **DIRECTED TISSUE GROWTH EMPLOYING REDUCED PRESSURE**
GEZIELTES GEWEBEWACHSTUM MIT VERRINGERTEM DRUCK
CROISSANCE DIRIGEE D'UN TISSU PAR PRESSION REDUITE

(30) Priority: 03.06.2002 US 161076
(43) Date of publication of application: 30.03.2005
(73) Proprietor: Wake Forest University Health Sciences, Winston-Salem, NC 27157 (US)
(72) Inventor: ARGENTA, Louis, C., Winston-Salem, NC 27104 (US); MORYKWAS, Michael, J., Pfafftown, NC 27040 (US); WEBB, Lawrence, X., Winston-Salem, NC 27106 (US)
(74) Representative: Naylor, Matthew John
(86) International application number: PCT/US2003/016763
(87) International publication number: WO 2003/101385

(56) References cited:
- EP-A- 1 064 958
- WO-A-94/20041
- WO-A-99/51164
- US-A- 5 717 030
- US-A1- 2001 043 943
- US-A1- 2001 043 943

## Description

### Field of the Invention

The present invention relates to an apparatus for promoting directed tissue growth, and more particularly to an apparatus for providing directed tissue growth within a host matrix through the application of reduced pressure to the tissue to be grown.

### Background of the Invention

Promoting the growth of tissue, especially tissue damaged through trauma or disease, has long been an area of concern in medical practice. Such damage or disease, including complications due to infection, may hinder or prevent healing of an injury due to a lack of healthy tissue growth. Many diseases and certain injuries involve affected tissue that cannot heal spontaneously. Such is the case, for example, for an open pilon fracture of bone tissue. Historically, a pilon fracture involves a high complication rate. Such complications include infection, nonunion, failure to obtain or maintain a reduction of the joint surface, and early and late arthritis. Under such conditions, failure to achieve sufficient healing of the pilon fracture could necessitate amputation.

In the 1970s and early 1980s the prescribed treatment for most pilon fracture injuries was open reduction and internal fixation, usually with a metaphysical bone graft. Reports of high complication rates with this approach prompted many surgeons to use indirect methods such as bridging external fixation and to limit the surgery to what was necessary for the joint reduction. Awareness of the issues of timing has prompted some to use a staged procedure, with bridging external fixation initially, followed by open but limited surgery. The incisions are dictated by fracture patterns, and the timing is dictated by resolution of the soft tissue envelope.

However, despite these approaches, cases arise where a major complication, e.g., a deep infection, can develop. Depending on the patient's medical condition, such as the condition of local blood vessels, customary treatment by application of a free muscle flap may be inappropriate. In such instances, traditional treatment offers a poor prognosis for salvage of the affected tissue. In such cases, where there is a likelihood of an infected nonunion and its associated pain, deformity and poor function, amputation is the appropriate and preferred medical treatment. Thus, it could be a great advance to the medical practice to provide an apparatus to promote healthy bone tissue growth under such circumstances to avoid the drastic treatment of amputation.

As further example, diseases such as cancer often result in tissue damage that does not heal spontaneously, and treatment of such resulting tissue damage would benefit from an apparatus and method to promote tissue growth. For example, many patients who experience injuries or suffer from bone cancer require replacement of a missing piece of bone. Current techniques for bone replacement include: moving a piece of the bone from an uninjured site to the injured site; use of cadaver bone; or the use of metal rods or plates. These options are not always possible due to the potential for defect from the bone donor site, or the lack of availability of cadaver bone. Hence, growth of new healthy bone tissue would provide a valuable treatment option in such cases.

In addition to growth of bone tissue, growth of other body tissues such as cartilage, skin, tendon, nerves, breast-tissue, and organs such as the liver or pancreas, would provide a valuable advance in the medical practice. Many diseases exist which damage the tissue of an organ beyond the ability of the body to naturally repair such damage. For example, chronic injury to the liver through viral infection or other causes can ultimately lead to cirrhosis of the liver. As cirrhosis progresses healthy tissue is replaced with fibrous tissue. The blood vessels thicken and their channels may become obliterated, which reduces blood flow in the organ. The normal structure of the internal tissue is lost, and only nonfunctioning scar tissue remains. The lack of healthy tissue eventually leads to death. It would be a great advance to promote growth of remaining healthy tissue in the liver in combination with treatment for the underlying cause of the cirrhosis.

Moreover, many other diseases exist which are caused by damage to tissues of an organ, of which, diabetes is one of pressing importance. Presently, the number of individuals with diabetes doubles every 15 years. While insulin treatment can prevent early death from diabetic coma, such treatment does not prevent the chronic, disabling complications of the disease. Currently, diabetes mellitus is among the top 10 causes of death in the United States, and is the leading cause of blindness and uremia.

The first type of diabetes, Type I, is caused by failure of the pancreas to secrete insulin. Normally, insulin is synthesized in the beta cells of the islets of Langerhans of the pancreas. Individuals affected with Type I diabetes have insulin deficiency due to islet cell loss. Recent medical studies have shown the transplantation of cadaver beta cells to a diabetic patient can provide the pancreas with the ability to produce insulin. However, such an approach is limited due to the lack of availability of cadaver donor cells, the need for subsequent transplants, as well as complications and side effects commonly encountered in transplantation, such as the need for continued use of antirejection drugs. The ability to provide directed tissue growth of beta cells within the pancreas, or externally to the pancreas for transplantation into the pancreas, would therefore be a significant advance the treatment of diseases such as diabetes.

US2001/0043943 proposes a wound care bandage including a structure to provide a vacuum space. WO99/51164 proposes a method of tissue reconstruction in which an injection means is positioned beneath a soft tissue defect and biocompatible material is injected into a subcutaneous location to treat the defect. WO94/20041 proposes a method of treating tissue damage comprising applying a negative pressure to a wound.

### Summary of the Invention

The present invention provides an apparatus for growing a tissue according to claim 1.

In accordance with the present invention a directed tissue growth apparatus is provided for growing tissue by applying a tissue growth medium (including a porous bone substitute material) to the tissue and applying a reduced, sub-atmospheric pressure at the growth medium and the tissue in a controlled manner for a selected time period. The application of reduced pressure at the growth medium and tissue promotes growth of the tissue within the tissue growth medium, which provides benefits such as accelerated healing rates and replacement of missing, diseased, or damaged tissue. Tissues that may exhibit a positive response to treatment by the application of reduced pressure include bone, cartilage, tendon, nerves, skin, breast tissue, and organs such as the liver or pancreas, for example.

The directed tissue growth apparatus in accordance with the present invention includes a reduced pressure application appliance which is applied to a treatment site where tissue is to be grown. Such a treatment site may be located in vivo or in vitro. The reduced pressure application appliance includes a tissue growth medium (including a porous bone substitute material) for placement in contact with the tissue to be grown to provide a medium into which cells may be grown. The form of the tissue growth medium is selected with regard to the type of tissue to be grown. For example, the tissue growth medium may comprise a layer of material suitable for use as artificial skin to replace damaged or missing skin. In such a case, the tissue growth medium may conveniently comprise a bioabsorbable material. A bioabsorbable material is a material that may dissolve in the tissue or which may be incorporated in the tissue as a substantially indistinguishable component. The tissue growth medium may also comprise a porous scaffold or matrix, which may include one or more of a naturally occurring fibrous or fibrous-proteinaceous material, such as collagen, or a synthetic resorbable material. For example, the scaffold may comprise the bone substitute material, such as a bioglass or ceramic. Porous materials permit growth of cells within the pores of the material and permit gas flow to the tissue during times of non-application of reduced pressure. In addition, during times when the sub-atmospheric pressure is applied to such porous materials, the porous materials facilitate distribution of sub-atmospheric pressure and fluid flow.

Optionally, an open-cell foam section, for connection to a source of reduced pressure, may be provided in contact with a selected tissue growth medium to apply a reduced pressure to the tissue growth medium. For example, the open-cell foam section may desirably be used with a particular tissue growth medium such as artificial skin, to better apply and distribute sub-atmospheric pressure across the skin surface.

The appliance also includes a cover for covering and enclosing the tissue and the tissue growth medium. The cover also functions to cover and enclose the open-cell foam section, when used. In applications where the cover is not self-sealing by suction, the appliance may also include sealing means for sealing the cover to the region surrounding the tissue to be grown in order to maintain reduced pressure in the vicinity of the tissue during tissue growth. When the cover is sealed in position over the tissue site, a generally fluid-tight or gas-tight sealed enclosure is formed over or about the tissue site. The sealing means may be in the form of an adhesive applied to the underside of the cover or at least to the periphery of the underside of the cover for sealing the cover to the region surrounding the tissue. The sealing means may also include a separate sealing member, such as an adhesive strip, a sealing ring, a tubular pad or an inflatable cuff, for use with the cover for positioning around the region surrounding the tissue to be grown. In selected embodiments, the reduced pressure within the sealed enclosure beneath the cover may serve to self adhere and seal the cover in position at the tissue growth site. The reduced pressure appliance may also include a suction port for enabling reduced pressure to be supplied within the sealed volume enclosed beneath the cover. The suction port may be in the form of a nipple on the cover. Alternatively, the suction port may be in the form of a tube attached to the cover. As yet another alternative, the port may be provided at the mouth of a suction tube that is inserted beneath the cover.

A vacuum system is connected with the reduced pressure appliance in order to provide suction or reduced pressure to the appliance. For this purpose, the vacuum system includes a suction pump or suction device for connection with the suction port of the appliance for producing the reduced pressure over the tissue site. The vacuum system may include a section of hose or tube, such as a vacuum hose, that interconnects the suction device with the suction port of the appliance to provide the reduced pressure at the tissue site. A tube of the appliance may serve as the vacuum hose for connection with the suction device. Further, a mouth of the suction hose may serve as the suction port in applications where the suction hose is not connected to a separate port.

A collection device in the form of a fluid trap may be provided intermediate the vacuum hose of the suction device and the suction port of the appliance to trap any exudate which may be aspirated from the tissue by the reduced pressure appliance. A stop mechanism may also be provided for the vacuum system to halt application of the reduced pressure at the tissue site in the event that a predetermined quantity of exudate has been collected. The apparatus may also include a control device for controlling the pump and for providing intermittent or cyclic production of reduced pressure.

In a particular embodiment of the invention, the cover for the reduced pressure appliance may be in the form of a gas impermeable covering sheet of flexible polymer material, such as polyurethane, having an adhesive backing that provides the seal for securing the sheet over the tissue site to provide a gas-tight or fluid-tight sealed enclosure over the tissue site. A semi-permeable cover may also be used in selected applications. The vacuum system of the tissue treatment apparatus may include a suction pump having a vacuum hose that is connected with or, alternatively, integral with, a suction tube serving as a suction port for the appliance. The suction tube for the appliance runs beneath the cover sheet when sealed in position over the tissue site and into the fluid-tight enclosure provided at the tissue site beneath the cover sheet. An adhesive backing on the cover sheet is used to provide a fluid-tight seal around the feedthrough for the suction tube at the tissue site. Within the enclosure, the suction tube is connected to a section of open-cell foam in communication with the tissue growth medium into which the tissue may be grown. The open-cell foam functions to more uniformly apply reduced pressure or suction over the tissue growth medium. Likewise, the open cell foam section communicates the reduced pressure to the tissue site under the cover sheet while holding the cover sheet substantially out of contact with the tissue during the application of reduced pressure at the enclosed tissue site. The tissue growth medium comprises a porous bone substitute material. In such a case, the suction tube may be connected directly with the tissue growth medium, and the open-cell foam section may be omitted.

### Brief Description of the Drawings

The foregoing summary, as well as the following detailed description of the preferred embodiments of the present invention, will be better understood when read in conjunction with the appended drawings, in which:

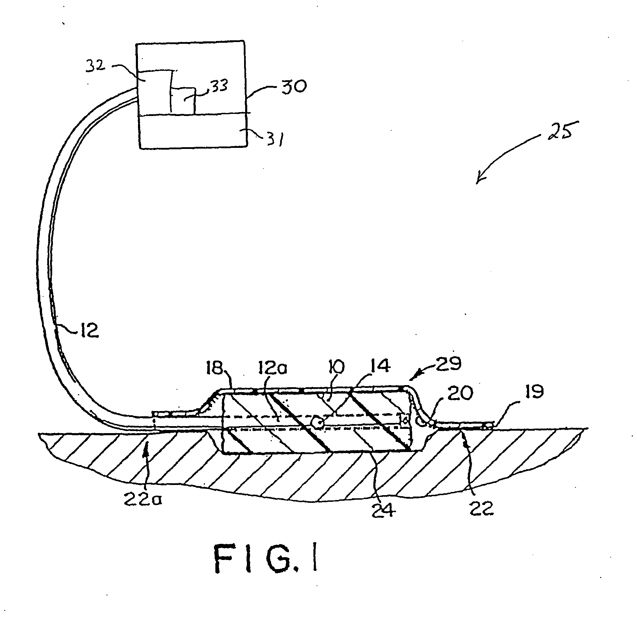

Figure 1 is a schematic cross-sectional view of a reduced pressure appliance comprising a porous tissue growth medium, a flexible hose for connecting the tissue growth medium with a vacuum system, and an adhesive-backed flexible polymer sheet overlying the growth medium to provide a seal over a tissue to be grown; and

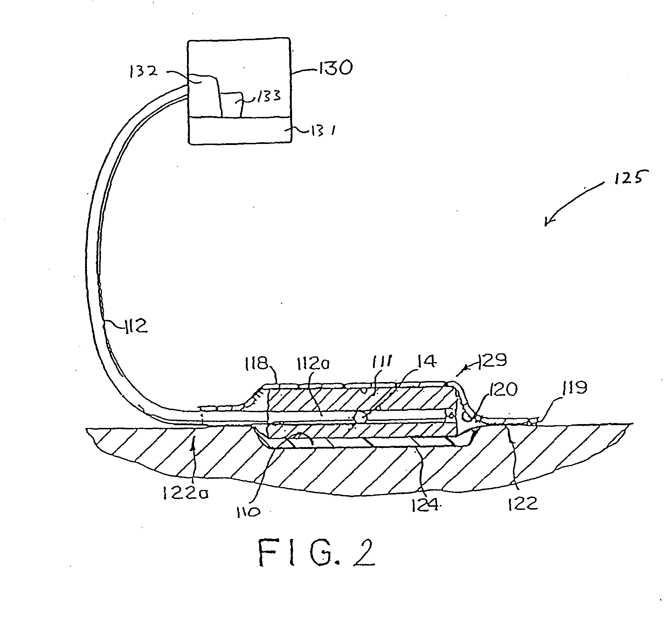

Figure 2 is a schematic cross-sectional view of a reduced pressure appliance comprising a foam section in communication with a tissue growth medium, a flexible hose for connecting the foam section with a vacuum system, and an adhesive-backed flexible polymer sheet overlying the growth medium-foam section assembly to provide a seal over a tissue to be grown.

### Detailed Description of the Preferred Embodiments

In accordance with the present invention, a directed tissue growth apparatus is provided for promoting growth in a tissue by application of reduced pressure (i.e., below atmospheric pressure) so that suction may be applied to a tissue site in a controlled manner for a selected time period.

Referring to Fig. 1, a directed tissue growth apparatus, generally designated 25, is depicted having a reduced pressure appliance 29 for enclosing a tissue site to provide a fluid-tight or gas-tight enclosure over the tissue site to grow tissue 24 in a tissue growth medium 10 through the application of sub-atmospheric pressure. The directed tissue growth apparatus 25 includes a reduced pressure appliance, generally designated 29, which is applied to and sealed over a tissue site in order to enclose the tissue site to form a reduced pressure chamber about the tissue site for treatment with suction or reduced pressure within a sealed generally fluid-tight or gas-tight enclosure. For the purpose of creating suction within the appliance 29, the appliance 29 is connected with a vacuum system, generally designated 30, to provide a source of suction or reduced pressure for the sealed appliance 29 at the tissue site. The vacuum system 30 may include a suction device 31 and an optional fluid collection device 32 intermediate the hose 12 and suction device 31. The fluid collection device 32 functions to collect any exudate that may be aspirated from the tissue. A stop mechanism 33 may be provided to halt application of the suction device 31 upon collection of a predetermined quantity of fluid in the fluid collection device 32. The appliance 29 includes a fluid-impermeable tissue cover 18 in the form of a flexible, adhesive, fluid impermeable polymer sheet for covering and enclosing the tissue 24 at the tissue site. The tissue cover 18 includes an adhesive backing 20 which functions to seal the tissue cover about the periphery of the tissue 24 to provide a generally gas-tight or fluid-tight enclosure over the tissue 24. The adhesive cover sheet 18 must have sufficient adhesion to form a fluid-tight or gas-tight seal 19 around the tissue 24 and to hold the sheet 18 in sealed contact at the attachment site during the application of suction or reduced pressure. Alternatively, the cover 18 may be provided in the form of a rigid or semi-rigid cover adapted to form a fluid-tight or gas-tight seal around the tissue. Suitable modifications may be made to the appliance to provide a reduced pressure chamber for treating the selected tissue.

The appliance 29 also includes a porous tissue growth medium 10 which is positioned under the cover 18 on or in the tissue 24. The tissue growth medium 10 is disposed over a sufficient expanse of the tissue 24 to promote sufficient growth of new tissue cells within the tissue growth medium 10. The tissue growth medium 10 should be sufficiently porous to allow for connection to a vacuum system 30 to supply sub-atmospheric pressure to the tissue 24 and the tissue growth medium 10. The tissue growth medium 10 may also be perforated to enhance gas flow and to reduce the weight of the appliance. The configuration and composition of the tissue growth medium 10 can be adjusted to suit the particular tissue type. For example, for growth in bone tissue, the tissue growth medium 10 may comprise a natural, synthetic, or natural-synthetic hybrid porous material. Such a tissue growth medium 10 may conveniently be chosen to provide a scaffold to support or direct osteoconduction, i.e. bone formation. Alternatively or additionally, such a tissue growth medium 10 may be selected from materials which induce differentiation of stem cells to osteogenic cells, i.e. osteoinductive agents, or materials which provide stem cells, e.g. bone marrow aspirate.

For example, a tissue growth medium 10 for use in bone growth may be a bioglass, ceramic material, or other natural or synthetic porous material. In particular, materials comprising calcium sulphate or calcium phosphate are suited for use as a bone tissue growth medium 10. A calcium sulfate bone substitute is completely absorbed by osteoclasts, and osteoblasts will attach to the calcium sulfate bone substitute and lay osteoid on it. The process of absorption by osteoclasts is complete and quick. Hence, it can be used with antibiotics in presence of infection. A chief advantage is that a calcium sulfate bone substitute can be used in presence of infection as well as being one of the least expensive bone substitutes. Calcium sulphate bone substitutes principally possess osteoconductive properties and no osteoinductive properties, though such a material could be modified to provide osteoinductive properties. One suitable calcium sulphate bone substitute is OSTEOSET® Bone Graft Substitute, a product of Wright Medical Technology, Inc. of Arlington TN.

Another class of suitable materials is one comprising various derivates of calcium phosphate, which can be used to provide a structural matrix for osteoconduction. These derivatives also do not possess any osteoinductive properties. The commonly used derivates are: hydroxyapatite (coral based or chemically derived synthetic ceramic), fluorapatite, tri-calcium phosphate, bioglass ceramics and combinations thereof. One suitable calcium phosphate bone substitute is OsteoGraft^{™} Bone Graft Substitute, a product of Millenium Biologix of Kingston, Ontario, Canada.

The appliance 29 also includes a suction port in the form of a hollow suction tube 12 that connects either directly or indirectly with the vacuum system 30 to provide suction within the sealed enclosure. The outlet of suction tubing 12 serves as a suction port for the appliance 29. An end segment 12a of the tubing 12 is embedded within the tissue growth medium 10 for providing suction or reduced pressure within the chamber provided under the tissue cover 18. The open-cell structure of the tissue growth medium 10 also permits the tissue growth medium 10 to distribute the reduced pressure within the chamber. Embedding the open end of segment 12a of tubing 12 within the interior of the tissue growth medium 10 permits the tissue growth medium 10 to function as a shield to help prevent the tissue cover 18 from being inadvertently sucked into occlusive engagement with the open end of the tube thereby plugging the tube 12 and restricting gas flow. The open-cell structure of the tissue growth medium 10 also permits the tissue growth medium 10 to distribute the reduced pressure within the sealed enclosure.

The tube segment 12a embedded within the tissue growth medium 10 optionally has at least one side port 14 for positioning within the interior of the tissue growth medium 10 to promote substantially uniform application of reduced pressure throughout the enclosure. Positioning the side port 14 of tube segment 12a within the interior of the tissue growth medium 10 permits the tissue growth medium 10 to also function as a shield for the side port to thereby prevent the tissue cover 18 from being sucked into the side port 14 and thereby restricting gas flow. The open cells of the tissue growth medium 10 facilitate growth of tissue therein and facilitate gas flow throughout the enclosure. In addition, the tissue growth medium 10 functions to hold the tissue cover 18 generally out of contact with the tissue 24 during the application of suction within the enclosure.

Tubing 12 and tube segment 12a may be sufficiently flexible to permit movement of the tubing but are sufficiently rigid to resist constriction when reduced pressure is supplied to the appliance 29 or when the location of the tissue is such that the patient must sit or lie upon the tubing 12 or upon the reduced pressure appliance 29. The assembly comprising the tissue growth medium 10 and the tube 12 may be fabricated by snaking the end of the tube segment 12a through an internal passageway in the tissue growth medium 10 such as by pulling the end of the tube segment 12a through the passageway using forceps. The assembly is preferably prepared prior to use under sterile conditions and then stored in an aseptic package.

In order to use the reduced pressure appliance 29 at the site of the tissue 24, the flexible, fluid-impermeable, adhesive tissue cover sheet 18 is secured in position at the tissue site, overlying the tissue growth medium 10 disposed in contact with the tissue 24. The tissue cover sheet 18 is secured and sealed to the attachment site 22 by an adhesive layer 20 on the under surface of the tissue cover 18 to form a gas-tight seal 19 about the tissue 24. The tissue cover 18 also provides a gas-tight seal around the tubing 12 at the feedthrough location 22a where the tubing 12 emerges from beneath the tissue cover 18. The tissue cover 18 is preferably formed of a fluid impermeable or gas impermeable flexible adhesive sheet such as Ioban, a product of the 3M Corporation of Minneapolis, MN.

Predetermined amounts of suction or reduced pressure may be produced by the vacuum system 30. The vacuum system 30 is preferably controlled by a control device or control circuitry that includes a switch or a timer which may be set to provide cyclic on/off operation of the vacuum system 30 according to user-selected intervals. Alternatively, the vacuum system 30 may be operated continuously without the use of a cyclical timer. The control may also include a pressure selector to enable the amount of suction produced by the system to be adjusted so that a suitable sub-atmospheric pressure may be created within the chamber. Operation of the vacuum system 30 may be controlled to permit graduated increases in the amount of vacuum applied or graduated decreases in the amount of vacuum applied.

Referring to Fig. 2, an alternative configuration of the reduced pressure appliance 129 is shown which is similar to the reduced pressure appliance 29 of Fig. 1. The elements of the appliance 129 of Fig. 2 which are similar to like elements depicted in Fig. 1 utilize the same reference number as used in Fig. 1 but with a 100-series added to such reference numerals. A principal difference between the reduced pressure appliance 129 of Fig. 2 and that of Fig. 1 is that a porous open-cell foam section 111 is provided for communication with the vacuum system 130. The open-cell foam section 111 in turn communicates with the tissue growth medium 110, which is porous.

The directed tissue growth apparatus 125 includes a reduced pressure appliance, generally designated 129, which is applied to and sealed over a tissue site in order to enclose the tissue site for treatment with suction or reduced pressure within a sealed generally fluid-tight or gas-tight enclosure. For the purpose of creating suction within the appliance 129, the appliance 129 is connected with a vacuum system, generally designated 130, to provide a source of suction or reduced pressure for the sealed appliance 129 at the tissue site. The appliance 129 includes a fluid-impermeable tissue cover 118 in the form of a flexible, fluid impermeable polymer sheet for covering and enclosing the tissue 124 at the tissue site. The tissue cover 118 may include an adhesive backing 120 at least around the periphery of the cover which functions to seal the tissue cover proximate to the tissue 124 to provide a generally gas-tight or fluid-tight enclosure over the tissue 124. The adhesive cover sheet 118 must have sufficient adhesion to form a fluid-tight or gas-tight seal 119 around the tissue 124 and to hold the sheet 118 in sealed contact with the attachment site around the tissue 124 during the application of suction or reduced pressure. The cover 118 may also be provided in the form of a rigid or semi-rigid cover which cooperates with a suitable seal to form a fluid-tight or gas-tight seal around the tissue.

The appliance 129 also includes a porous open-cell foam section 111 which is placed under the cover 118 and in direct or indirect contact with a tissue growth medium 110. The open-cell foam section 111 should be sufficiently porous to allow for connection to the vacuum system 130 to transmit sub-atmospheric pressure to the tissue 124 and the tissue growth medium 110. The tissue growth medium 110 is placed over a sufficient expanse of the tissue 124 to promote sufficient growth of new tissue cells within the tissue growth medium 110. The tissue growth medium 110 and/or the foam section 111 may also be perforated or channeled to enhance gas flow and to reduce the weight of the appliance. In a comparative application, the configuration depicted in Fig. 2 is particularly suitable for use with non-porous growth media or a relatively thin growth medium, such as a skin substitute material. A skin substitute material may comprise a multilayer structure having, for example, a layer of collagen for contact with the tissue to be grown and a silicone layer disposed on top of the collagen layer for contact with the foam section 111. A suitable skin substitute material is INTEGRA^{®} Dermal Regeneration Template, a product of Integra LifeSciences Corp. of Plainsboro, NJ. The silicone layer provides a removable backing to support the collagen layer during tissue ingrowth. After growth has continued to a desired stage, the silicone layer may be removed from the collagen layer, leaving the neodermis in place, onto which a thin split thickness skin graft may be placed.

For another comparative application, the growth medium 110 can be formed for the purpose of growing an organ, such as the pancreas or liver. The growth medium 110 may take the form of a scaffold/matrix of either a naturally occurring molecule (e.g., collagen) or of resorbable materials (e.g., polyglycolic acid or polygalactic acid or a combination thereof). The growth medium 110 may be formed from commercially available screens which are layered to the desired thickness.

The appliance 129 also includes a suction port in the form of a hollow suction tube 112, similar to the tube 12 of Fig. 1, that connects with the vacuum system 130 to provide suction within the sealed enclosure. The suction tubing 112 provides at least one suction port for the appliance 129. Unlike the device of Fig. 1, an end segment 112a of the tubing 112 is embedded within the foam section 111, rather than in the tissue growth material, for providing suction or reduced pressure within the enclosure provided under the tissue cover 118. The open-cell structure of the foam section 111 permits the foam section 111 to distribute the reduced pressure within the enclosure. Embedding the open end of segment 112a of tubing 112 within the interior of the foam section 111 permits the foam section 111 to function as a shield to help prevent the tissue cover 118 from being inadvertently sucked into the open end of the tube thereby plugging the tube 112 and restricting gas flow. The open-cell structure of the foam section 111 also permits the foam section 111 to distribute the pressure within the sealed enclosure.

The tube segment 112a embedded within the foam section 111 preferably has at least one side port 114 for positioning within the interior of the foam section 111 to promote substantially uniform application of reduced pressure throughout the enclosure. Positioning the side port 114 of tube segment 112a within the interior of the foam section 111 permits the foam section 111 to function as a shield for the side port to thereby prevent the tissue cover 118 from being sucked into the side port 114 and thereby restricting gas flow. The open cells of the foam section 111 facilitate gas flow throughout the enclosure. In addition, the foam section 111 functions to hold the tissue cover 118 generally out of contact with the tissue 124 during the application of suction within the enclosure.

Similar to the appliance 29 of Fig. 1, the flexible, fluid-impermeable, adhesive tissue cover sheet 118 of appliance 129 is secured, during use, in position at the tissue site, overlying the foam section 111 and tissue growth medium 110 which contacts the tissue 124. The tissue cover sheet 118 is secured and sealed to the attachment site 122 by an adhesive layer 120 on the under surface of the tissue cover 118 to form a gas-tight seal 119 about the tissue 124. The tissue cover 118 also provides a gas-tight seal around the tubing 112 at the feedthrough location 122a where the tubing 112 emerges from beneath the tissue cover 118.

Reduced pressure appliances are useful for growing a variety of tissues. Directed growth of a tissue can be carried out by securing a reduced pressure appliance to the treatment site as previously shown and described, and then maintaining a substantially continuous or cyclical reduced pressure within the appliance until the newly grown tissue has reached a desired degree of development. This may be done using a subatmospheric pressure ranging from about 51 to about 99 kPa (about 0.5 to about 0.98 atmospheres),and more specifically about 74 kPa to about 96 kPa (about .73 atmospheres to about .95 atmospheres), and more preferably using a subatmospheric pressure ranging between about 81 kPa to about 91 kPa (about 0.8 to about 0.9 atmospheres). The time period for use on a tissue may preferably be at least 48 hours, but can, for example, be extended for multiple days. Satisfactory growth of various types of tissues has been obtained via the use of reduced pressures equivalent to about 3.3 to 27kPa (1 to 8 in. Hg) below atmospheric pressure.

Supplying reduced pressure to the appliance in an intermittent or cyclic manner may be desirable for growing tissues. Intermittent or cyclic supply of reduced pressure to an appliance may be achieved by manual or automatic control of the vacuum system. A cycle ratio, the ratio of "on" time to "off" time, in such an intermittent reduced pressure treatment may be as low as 1:10 or as high as 10:1. The preferred ratio is approximately 5 minutes on which is usually accomplished in alternating intervals of 5 minutes of reduced pressure supply and 2 minutes of non-supply.

A suitable vacuum system includes any suction pump capable of providing at least 0.67 kPa (5 mm of Hg) of suction to the tissue, and preferably up to 17 kPa (125 mm of Hg) suction, and most preferably up to approximately 27 kPa (200 mm of Hg) of suction or even higher as necessary to achieve subatmospheric pressures of about 51 kPa (.5 atmospheres). The pump can be any ordinary suction pump suitable for medical purposes that is capable of providing the necessary suction. The dimension of the tubing interconnecting the pump and the reduced pressure appliance is controlled by the pump's ability to provide the suction level needed for operation. For example, a 6.4 mm (1/4 inch) diameter tube may be suitable.

Further features of the apparatus shall be made apparent in the following example.

### Comparative Example 1

This example was designed to demonstrate the effectiveness of the invention for accelerating the rate of vascular ingrowth in a skin substitute material.

Several 25 kg pigs were sedated and prepped for surgery. A series of 2 cm by 5 cm full thickness defects (down to the deep back muscles) were created on each side of the back of the animal. A directed tissue growth apparatus of the type shown in Fig. 2 comprising INTEGRA^{®} Dermal Regeneration Template was applied to the wound site on one side of each animal. An INTEGRA^{®} Dermal Regeneration Template was applied to the wound site on the other side of each animal. The INTEGRA^{®} Dermal Regeneration Template was applied according the manufacturer's directions. Vacuum (125 mm Hg, continuous) was applied to the directed tissue growth apparatus positioned on the selected wound sites on one side of the pigs. After 72 hours, the apparatus was removed, and the pieces of INTEGRA^{®} Dermal Regeneration Template were peeled off from the vacuum-treated and non-vacuum-treated wound sites at 10 cm/sec. The force required to pull the pieces off was recorded. A paired T-test was used to determine statistical significance of the pull force data.

The INTEGRA^{®} Dermal Regeneration Template at the non-vacuum-treated wound site required a force of 1.25 +/- 0.51 Newton to separate the template from the wound site. The INTEGRA^{®} Dermal Regeneration Template at the vacuum-treated wound site required a greater force of 2.25 +/- 0.51 Newton to separate the vacuum-treated template from the wound site. The increased pull force required at the vacuum-treated wound site indicated increased ingrowth of vasculature in the skin substitute material due to the vacuum treatment. The increased vascular ingrowth was confirmed by micrographs of the vacuum-treated and non-vacuum-treated pieces of INTEGRA^{®} Dermal Regeneration Template removed from the animals.

The terms and expressions which have been employed are used as terms of description and not of limitation and there is no intention in the use of such terms and expressions of excluding any equivalents of the features shown and described, or portions thereof, but it is recognized that various modifications are possible within the scope of the claimed invention.

## Claims

1. An apparatus (25; 125) for growing a tissue, comprising:
vacuum means (12, 30; 112, 130) for creating a sub-atmospheric pressure at a tissue to be grown (24; 124);
sealing means (29; 129) operatively associated with the vacuum means for maintaining the sub-atmospheric pressure at the tissue by contacting the region surrounding the tissue; and
a porous bone substitute material (10; 110) for positioning at the tissue within the sealing means and adapted to promote tissue growth therein.

2. An apparatus according to any one of the previous claims, wherein the bone substitute material comprises a bioabsorbable material.

3. An apparatus according to claim 1, wherein the bone substitute material comprises at least one of calcium sulfate, calcium phosphate, a bioglass, or a ceramic.

4. An apparatus according to any one of the previous claims, wherein the bone substitute material comprises a bioabsorbable scaffold material.

5. An apparatus according to any one of the previous claims, wherein the bone substitute material comprises a pore size sufficiently large to permit tissue growth therein.

6. An apparatus according to any one of the previous claims, comprising an open-cell foam section (111) in communication with the bone substitute material (110).

7. An apparatus according in claim 6, wherein the vacuum means includes a segment of tubing (112a) embedded within the foam section.

8. An apparatus according to any one of the previous claims, wherein the vacuum means includes a segment of tubing (12a) embedded within the bone substitute material.

9. An apparatus according to any one of the previous claims, comprising a flexible tube (12) having an inlet end inserted into the bone substitute material and an outlet end for extending from beneath the seal for supplying the sub-atmospheric pressure.

10. An apparatus according to any one of the previous claims, wherein the sealing means comprises a cover (18; 118) adapted to cover and enclose a tissue to be grown and adapted to maintain reduced pressure at the site of the tissue.

11. An apparatus according to claim 10, wherein the cover includes an adhesive material (20; 120) on the cover adapted to secure the cover to the tissue to be grown.

12. An apparatus according to claim 10 or 11, comprising a tubular member (12) having a first end inserted within a portion of the bone substitute material and having a second end extending from beneath the cover to a location external to the cover for supplying sub-atmospheric pressure beneath the cover.

13. An apparatus according to any one of the previous claims, wherein the sealing means comprises a fluid-impermeable cover (18; 118).

14. An apparatus according to any one of the previous claims, wherein the sealing means includes a flexible polymer sheet (18; 118) overlying the bone substitute material, the polymer sheet having adhesive (20; 120) on at least a surface facing the tissue to attach and seal the polymer sheet to the region surrounding the tissue.

15. An apparatus according to any one of the previous claims, wherein the sealing means includes a sealing rim in sealing contact with the region surrounding the tissue.

16. An apparatus according to any one of the previous claims, wherein the vacuum means supplies a sub-atmospheric pressure between 0.5 and 0.98 atmospheres to the tissue.

17. An apparatus according to any one of claims 1 to 15, wherein the vacuum means supplies a sub-atmospheric pressure between 0.73 and 0.95 atmospheres to the tissue.

18. An apparatus according to any one of claims 1 to 15, wherein the vacuum means supplies a sub-atmospheric pressure between 0.8 and 0.9 atmospheres to the tissue.

19. An apparatus according to any one of the previous claims, wherein the vacuum means operates continuously.

20. An apparatus according to any one of claims 1 to 18, wherein the vacuum means operates cyclically to provide periods of application and non-application of suction.

21. An apparatus according to claim 22, wherein the vacuum means provides periods of application and non-application of suction with the ratio of duration of application period to non-application period between 1:10 and 10:1.

22. An apparatus according to claim 21, wherein the duration of the application period is 5 minutes.

23. An apparatus according to claim 21, wherein the duration of the non-application period is 2 minutes.

## Patentansprüche

1. Vorrichtung (25; 125) zum Züchten von Gewebe, die Folgendes umfasst:
Vakuummittel (12, 30; 112, 130) um an dem Gewebe, das gezüchtet werden soll (24; 124), einen Unterdruck zu erzeugen;
Dichtungsmittel (29; 129), die operativ mit dem Vakuummittel verbunden sind, um den Unterdruck an dem Gewebe aufrecht zu erhalten, indem sie mit dem Bereich um das Gewebe in Kontakt stehen; und
ein poröses Knochenersatzmaterial (10; 110), zum Positionieren an dem Gewebe innerhalb der Dichtungsmittel, das geeignet ist, um Gewebewachstum in diesem zu fördern.

2. Vorrichtung nach einem der vorangegangenen Ansprüche, worin das Knochenersatzmaterial ein bioabsorbierbares Material umfasst.

3. Vorrichtung nach Anspruch 1, worin das Knochenersatzmaterial zumindest ein aus Calciumsulfat, Calciumphosphat, einem Bioglas oder einem Keramikmaterial ausgewähltes Material umfasst.

4. Vorrichtung nach einem der vorangegangenen Ansprüche, worin das Knochenersatzmaterial ein bioabsorbierbares Gerüstmaterial umfasst.

5. Vorrichtung nach einem der vorangegangenen Ansprüche, worin das Knochenersatzmaterial eine ausreichend große Porengröße aufweist, um Gewebewachstum in diesem zu ermöglichen.

6. Vorrichtung nach einem der vorangegangenen Ansprüche, worin sich ein Schaumabschnitt (111) mit offenen Zellen in Kommunikation mit dem Knochenersatzmaterial (110) befindet.

7. Vorrichtung nach Anspruch 6, worin das Vakuummittel ein Rohrsegment (112a) umfasst, das in dem Schaumabschnitt eingebettet ist.

8. Vorrichtung nach einem der vorangegangenen Ansprüche, worin das Vakuummittel einen Rohrabschnitt (12a) umfasst, der in dem Knochenersatzmaterial eingebettet ist.

9. Vorrichtung nach einem der vorangegangenen Ansprüche, die einen flexiblen Schlauch (12) aufweist, der ein in das Knochenersatzmaterial eingeführtes Einlassende und ein Auslassende aufweist, das sich unterhalb der Dichtung zur Zufuhr des Unterdrucks erstreckt.

10. Vorrichtung nach einem der vorangegangenen Ansprüche, worin das Dichtungsmittel eine Abdeckung (18; 118) umfasst, die geeignet ist, um ein Gewebe, das gezüchtet werden soll, abzudecken und einzuschließen, und geeignet ist, um den reduzierten Druck an der Stelle des Gewebes aufrecht zu erhalten.

11. Vorrichtung nach Anspruch 10, worin die Abdeckung ein Haftmaterial (20; 120) auf der Abdeckung umfasst, wobei das Haftmaterial ausgebildet ist, um die Abdeckung an dem Gewebe, das gezüchtet werden soll, fest anzulegen.

12. Vorrichtung nach Anspruch 10 oder 11, die ein röhrenförmiges Element (12) umfasst, das ein erstes Ende, das in einen Abschnitt des Knochenersatzmaterials eingeführt ist, und ein zweites Ende aufweist, das sich von unterhalb der Abdeckung zu einer Position außerhalb der Abdeckung erstreckt, um unterhalb der Abdeckung den Unterdruck aufrecht zu erhalten.

13. Vorrichtung nach einem der vorangegangenen Ansprüche, worin das Dichtungsmittel eine fluidundurchlässige Abdeckung (18; 118) umfasst.

14. Vorrichtung nach einem der vorangegangenen Ansprüche, worin das Dichtungsmittel eine flexible Polymerbahn (18; 118) umfasst, die über dem Knochenersatzmaterial liegt, wobei die Polymerbahn ein Haftmittel (20; 120) auf zumindest einer dem Gewebe zugewandten Oberfläche aufweist, um die Polymerbahn an dem das Gewebe umgebenden Bereich zu befestigen und diesen abzudichten.

15. Vorrichtung nach einem der vorangegangenen Ansprüche, worin das Dichtungsmittel einen Dichtungsrand umfasst, der sich in abdichtendem Kontakt mit dem das Gewebe umgebenden Bereich befindet.

16. Vorrichtung nach einem der vorangegangenen Ansprüche, worin das Vakuummittel dem Gewebe einen Unterdruck zwischen 0,5 und 0,98 Atmosphären zuführt.

17. Vorrichtung nach einem der Ansprüche 1 bis 15, worin das Vakuummittel dem Gewebe einen Unterdruck zwischen 0,73 und 0,95 Atmosphären zuführt.

18. Vorrichtung nach einem der Ansprüche 1 bis 15, worin das Vakuummittel dem Gewebe einen Unterdruck zwischen 0,8 und 0,9 Atmosphären zuführt.

19. Vorrichtung nach einem der vorangegangenen Ansprüche, worin das Vakuummittel kontinuierlich betrieben wird.

20. Vorrichtung nach einem der Ansprüche 1 bis 18, worin das Vakuummittel zyklisch betrieben wird, um Phasen bereitzustellen, in denen angesaugt wird, und solche, in denen nicht angesaugt wird.

21. Vorrichtung nach Anspruch 22, worin das Vakuummittel Phasen bereitstellt, in denen angesaugt wird, und solche, in denen nicht angesaugt wird, wobei das Verhältnis der Dauer der Ansaugphase zu der Dauer der Nicht-Ansaugphase zwischen 1:10 und 10:1 beträgt.

22. Vorrichtung nach Anspruch 21, worin die Dauer der Ansaugphase 5 min beträgt.

23. Vorrichtung nach Anspruch 21, worin die Dauer der Nicht-Ansaugphase 2 min beträgt.

## Revendications

1. Appareil (25; 125) pour la croissance d'un tissu, comprenant:
un moyen de vide (12, 30; 112, 130) pour créer une pression sous-atmosphérique à un tissu à faire croître (24; 124);
un moyen d'étanchéité (29; 129) fonctionnellement associé au moyen de vide pour maintenir la pression sous-atmosphérique au tissu en venant en contact avec la région entourant le tissu; et
un matériau de substitut d'os poreux (10; 110) pour le positionnement au tissu dans le moyen d'étanchéité et apte à encourager la croissance tissulaire dans celui-ci.

2. Appareil selon l'une quelconque des revendications précédentes, où le matériau de substitut d'os comprend un matériau bioabsorbable.

3. Appareil selon la revendication 1, où le matériau de substitut d'os comprend au moins un parmi le sulfate de calcium, le phosphate de calcium, un bioverre ou une céramique.

4. Appareil selon l'une quelconque des revendications précédentes, où le matériau de substitut d'os comprend un matériau de charpente bioabsorbable.

5. Appareil selon l'une quelconque des revendications précédentes, où le matériau de substitut d'os comprend une dimension de pore suffisamment grande pour permettre la croissance tissulaire dans celui-ci.

6. Appareil selon l'une quelconque des revendications précédentes, comprenant une section de mousse à alvéoles ouvertes (111) en communication avec le matériau de substitut d'os (110).

7. Appareil selon la revendication 6, où le moyen de vide comprend un segment de tube (112a) noyé dans la section de mousse.

8. Appareil selon l'une quelconque des revendications précédentes, où le moyen de vide comprend un segment de tube (12a) noyé dans le matériau de substitut d'os.

9. Appareil selon l'une quelconque des revendications précédentes, comprenant un tube flexible (12) ayant une extrémité d'entrée insérée dans le matériau de substitut d'os et une extrémité de sortie pour s'étendre depuis en dessous du joint pour réaliser la pression sous-atmosphérique.

10. Appareil selon l'une quelconque des revendications précédentes, où le moyen d'étanchéité comprend un couvercle (18; 118) apte à couvrir et à renfermer un tissu à faire croître et apte à maintenir une pression réduite au site du tissu.

11. Appareil selon la revendication 10, où le couvercle comprend un matériau adhésif (20; 120) sur le couvercle apte à fixer le couvercle au tissu à faire croître.

12. Appareil selon la revendication 10 ou 11, comprenant un élément tubulaire (12) ayant une première extrémité insérée dans une portion du matériau de substitut d'os et ayant une seconde extrémité s'étendant depuis en dessous du couvercle à un emplacement à l'extérieur du couvercle pour réaliser une pression sous-atmosphérique en dessous du couvercle.

13. Appareil selon l'une quelconque des revendications précédentes, où le moyen d'étanchéité comprend un couvercle imperméable au fluide (18; 118).

14. Appareil selon l'une quelconque des revendications précédentes, où le moyen d'étanchéité comprend une feuille de polymère flexible (18; 118) reposant sur le matériau de substitut d'os, la feuille de polymère ayant un adhésif (20; 120) sur au moins une surface orientée vers le tissu pour attacher et sceller la feuille de polymère à la région entourant le tissu.

15. Appareil selon l'une quelconque des revendications précédentes, où le moyen d'étanchéité comprend un bord d'étanchéité en contact étanche avec la région entourant le tissu.

16. Appareil selon l'une quelconque des revendications précédentes, où le moyen de vide fournit une pression sous-atmosphérique entre 0,5 et 0,98 atmosphères au tissu.

17. Appareil selon l'une quelconque des revendications 1 à 15, où le moyen de vide fournit une pression sous-atmosphérique entre 0,73 et 0,95 atmosphère au tissu.

18. Appareil selon l'une quelconque des revendications 1 à 15, où le moyen de vide fournit une pression sous-atmosphérique entre 0,8 et 0,9 atmosphère au tissu.

19. Appareil selon l'une quelconque des revendications précédentes, où le moyen de vide fonctionne continuellement.

20. Appareil selon l'une quelconque des revendications 1 à 18, où le moyen de vide fonctionne d'une manière cyclique pour réaliser des périodes d'application et de non-application d'aspiration.

21. Appareil selon la revendication 22, où le moyen de vide réalise des périodes d'application et de non-application d'aspiration, le rapport de la durée de la période d'application à la période de non-application étant entre 1:10 et 10:1.

22. Appareil selon la revendication 21, où la durée de la période d'application est de 5 minutes.

23. Appareil selon la revendication 21, où la durée de la période de non-application est de 2 minutes.
